# EUROPÄISCHE PATENTANMELDUNG

(11) **EP 2 193 796 A1**
(43) Veröffentlichungstag der Anmeldung: **09.06.2010**
(21) Anmeldenummer: 10158215.3
(22) Anmeldetag: 26.06.2002
(51) Int. Cl.: A61K 31/714, A61P 17/00

(54) **Verwendung von Corrinoiden zur Anwendung bei Hauterkrankungen**

(30) Priorität: 28.06.2001 DE 10130846
(62) Teilanmeldung aus: 02751075.9
(71) Anmelder: Regeneratio Pharma GmbH, 42857 Remscheid (DE)
(72) Erfinder: Klingelhöller, Karsten, 42105, Wuppertal (DE)
(74) Vertreter: Christophersen & Partner

(57) **Zusammenfassung**

Es wird die Verwendung von Verbindungen mit der Formel I 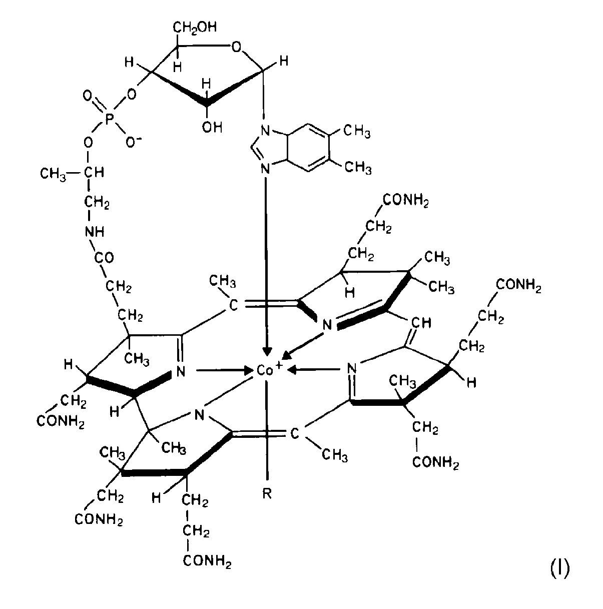 in der R für CN, OH, CH₃ oder H₂O stehen, in der Prophylaxe und zur Behandlung von allergischen Hautreaktionen beansprucht.

## Beschreibung

Die vorliegende Anmeldung ist eine Teilanmeldung aus der europäischen Patentanmeldung 02 751 075.9.

Die Erfindung betrifft eine neue Verwendung von Corrinoiden zur Behandlung von akuten und chronischen Hauterkrankungen bzw. -schäden und zur Prophylaxe von bestimmten Hauterkrankungen.

Die Behandlung von Hauterkrankungen, insbesondere von chronischen Hauterkrankungen, stellt in der Medizin ein großes Problem dar, da sie nur sehr begrenzt heilbar sind. Die Behandlung dieser Erkrankungen verschafft in vielen Fällen den Patienten nur eine geringe Linderung, in vielen Fällen ist gar kein Heilungserfolg zu beobachten. Hinzukommt, dass eine Vielzahl der eingesetzten Wirkstoffe wie z. B. Kortison starke Nebenwirkungen aufweisen.

Wegen der starken Nebenwirkungen der eingesetzten Wirkstoffe werden sie nur in akuten Fällen eingesetzt. Eine Prophylaxe bei Prädisposition für chronische Hauterkrankungen, wie es insbesondere bei Neurodermitis und Psoriasis der Fall ist, gibt es derzeit nicht.

Auch der Prophylaxe von anderen als chronischen Hauterkrankungen und möglichen Hautschäden sowie der Hautpflege wird große Aufmerksamkeit geschenkt. Dazu werden Hautpflegeprodukte und entsprechende pharmazeutische Zubereitungen eingesetzt, die unterschiedliche Inhalts- und Wirkstoffe enthalten, wie z.B. feuchtigkeitsspendende Substanzen, glättende Substanzen etc.

Für die Behandlung von Hautschäden, z. B. Verbrennungen, sind kühlende Gele im Handel erhältlich, die im Wesentlichen Linderung der Schmerzzustände verschaffen, in der Regel aber nicht den Heilungsprozeß der Haut unterstützen.

Seit man einen Zusammenhang zwischen intensiver Sonnenbestrahlung und Bildung von Hautkrebs festgestellt hat, schützen sich viele Menschen vor all zu großer Sonneneinwirkung durch sogenannte Sonnenschutzmittel, die UV-Filter beziehungsweise -Absorber enthalten. Die UV-Absorber oder UV-Schutzfilter sind organische Substanzen, welche in der Lage sind, elektromagnetische Strahlung im UV-Bereich zu absorbieren. Die Moleküle gehen hierbei in einen energetisch angeregten Zustand über. Beim Rückgang in den Grundzustand wird die aufgenommene Energie wieder vollständig, allerdings in einer für die Haut weniger oder nicht schädlichen Form wieder abgegeben, z.B. in Form von Wärme oder Licht größerer Wellenlänge und geringerer Frequenz.

Ein weiteres Gebiet der Behandlung von Hautschäden betrifft Wundheilungsstörungen und Narbenbildung. Wundheilungsstörung, d.h. der verzögerte bzw. gestörte Heilungsprozess von offenen Wunden ist häufig mit großen Unannehmlichkeiten verbunden, da offene Wunden schmerzhaft sein und sich auch entzünden können. Auch die bei normaler Wundheilung zurückbleibenden Narben sind aus ästhetischen Gründen unerwünscht, insbesondere, wenn si sich auf Hautflächen befinden, die in der Regel nicht von der Kleidung bedeckt sind, wie im Gesicht und auf den Händen.

Aufgabe der vorliegenden Erfindung ist es, einen wirksamen Wirkstoff zu finden, der zur Prophylaxe bei Disposition für chronische Hauterkrankungen, zur Prophylaxe und Behandlung von allergischen Hautreaktionen, wie z. B. bei Sonnenallergien (Dermatitis solaris, Dermatitis vernalis aurium, Dermatitis bullosa pratensis); und zur Behandlung von akuten Hautschäden, z. B. bei Verbrennungen (Combustio) einschließlich Sonnenbrand und anderen Strahlenschäden (Phototoxische Reaktionen, Photoallergische Reaktionen, Hautschäden durch ionisierende Strahlen (akute und chronische Radiodermatitis)) sowie zur Behandlung von Hautschäden, wie Wunden und der sich daraus bildenden Narben sowie bei Striae cutis bzw. distensae und Hautalterung, wie Faltenbildung einschließlich Cellulitis, wie Kälteschäden der Haut (Congelatio), Hautschäden durch Einwirken von Säuren und Laugen, Phakomatosen (Morbus Recklinghausen), Dermatomykosen, Polymorphe Lichtdermatosen, Ichthyosen und bei der Behandlung von entarteten Zellen, wie bei Cancerosen, gutartigen und/oder bösartigen Tumoren, wirksam ist und der möglichst keine Nebenwirkungen zeigt. Insbesondere für die Prophylaxe bei chronischen Hauterkrankungen, die möglichst im Säuglingsalter erfolgen sollte, ist es erforderlich, dass der Wirkstoff keine Nebenwirkungen zeigt.

Die vorliegende Erfindung betrifft die Verwendung von Corrinoiden mit der Formel I 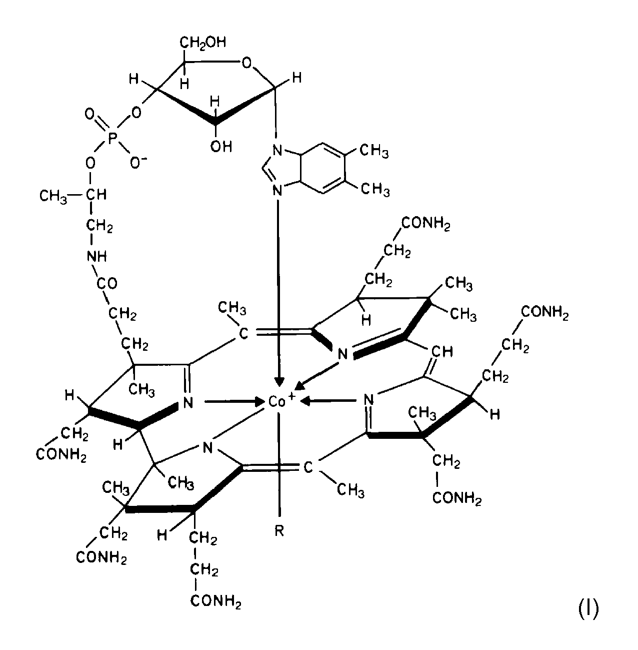 in der
R für CN, OH, CH₃ oder H₂O stehen,
zum Aufbau und Verbesserung der Widerstandsfähigkeit der Haut.

Die Corrinoide mit der Formel I sind auch unter der Bezeichnung Cobalamine oder Vitamin B₁₂ bekannt. Sie werden in der Medizin z. B. zur Behandlung von perniziöser Anämie oral, subkutan oder intravenös, in Form von Tabletten bzw. als Injektionslösung verabreicht. Im europäischen Patent 705 102 wird ihre Verwendung zur Behandlung von Hauterkrankungen offenbart. Als weitere für die erfindungsgemäße Verwendung geeigneten Derivate können neben den Cobalaminen auch die Cobamide, Cobamsäuren, Cobinamide, Cobinsäuren, Cobyrsäuren und Cobyrinsäuren genannt werden.

Vorzugsweise werden Verbindungen mit der Formel I zur Prophylaxe von chronischen Hauterkrankungen, insbesondere von entzündlichen und hyperproliferativen Hauterkrankungen bzw. kutanen Manifestationen immunologisch bedingter Erkrankungen, z.B. von: Psoriasis, atopischer Dermatitis, Kontakt-Dermatitis und anderen ekzematösen Dermatitiden, seborrhöischer Dermatitis, Neurodermitis, Decubitus, Lichen planus, Pemphigus, bullosem Pemphigoid, Epidermolysis bullosa, Urticaria, Angioödemen, Vasculitiden, Erythemen, kutanen Eosinophilien, Lupus erythematodes sowie von Alopecia areata eingesetzt.

Ein weiterer Gegenstand der vorliegenden Erfindung ist die Verwendung der Verbindungen mit der oben dargestellten Formel zur Prophylaxe und Behandlung von allergischen Hautreaktionen, wie z. B. bei Sonnenallergien (Dermatitis solaris, Dermatitis vernalis aurium, Dermatitis bullosa pratensis), Lebensmittelallergien z.B. Allergien ausgelöst durch Bambussprossen, bittere Mandeln, Bohnen, Austern und Datteln.

Noch ein weiterer Gegenstand der vorliegenden Erfindung ist die Verwendung der Verbindungen mit der oben dargestellten Formel zur Behandlung von akuten Hautschäden, z. B. bei Verbrennungen (Phototoxische Reaktionen, Photoallergische Reaktionen, Hautschäden durch ionisierende Strahlen (akute und chronische Radiodermatitis)) einschließlich Sonnenbrand und anderen Strahlenschäden sowie zur Behandlung von Hautschäden, wie Wunden und der sich daraus bildenden Narben sowie bei Striae cutis bzw. distensae und Hautalterung, wie Faltenbildung einschließlich Cellulitis, sowie Kälteschäden der Haut (Congelatio), Hautschäden durch Einwirken von Säuren und Laugen, Phakomatosen (Morbus Recklinghausen), Dermatomykosen, Polymorphe Lichtdermatosen, Ichthyosen und bei der Behandlung von entarteten Zellen, wie bei Cancerosen, gutartigen und/oder bösartigen Tumoren.

Im Rahmen der vorliegenden Erfindung ist zwischen der Behandlung von bereits aufgetretenen Erkrankungen und der Prophylaxe bei einer entsprechenden Prädisposition zu unterscheiden. Die Behandlung von Erkrankungen bedeutet, dass bei deren Manifestation eine Behandlung erfolgt, die bei chronischen Erkrankungen in der Regel fortdauernd ist und bei vorübergehend auftretenden Erkrankungen nur während deren Auftretens.

Prophylaxe bedeutet, dass dem Auftreten von Erkrankungen vorgebeugt werden soll. Besteht bei einem Säugling beispielsweise eine Prädisposition für chronische Hauterkrankungen wie Psoriasis oder Neurodermitis, so kann erfindungsgemäß eine pharmazeutische Zubereitung, die die oben wiedergegebene Verbindung enthält vorbeugend eingesetzt werden. Es wurde festgestellt, dass bei vorbeugend behandelten Säuglingen im Kleinkindalter, in dem derartige Hauterkrankungen üblicherweise zum Ausbruch kommen, keine bzw. nur eine sehr schwache und gegenüber den ebenfalls erkrankten Elternteilen deutlich verringerte Form der Erkrankung auftrat.

Die erfindungsgemäß eingesetzten Verbindungen mit der Formel I können in einer beliebigen pharmazeutischen Darreichungsform verabreicht werden. Für die erfindungsgemäße Verwendung werden die Verbindungen mit der Formel I vorzugsweise topisch verabreicht. Vorzugsweise werden die Verbindungen Kombination mit in der pharmazeutischen Industrie für topische Anwendungen üblichen Träger- bzw. Verdünnungstoffen eingesetzt werden.

Für die obige Verwendung hängt die zu verabreichende Dosis von der verwendeten Verbindung, der Verabreichungsart sowie der Behandlungsart ab. Es werden sehr gute Ergebnisse erhalten, wenn eine oder mehrere Verbindungen mit der Formel I mit einer Konzentration von 1 x 10⁻⁴ bis 1 Gew.-% einmal oder mehrmals täglich lokal verabreicht werden.

Die Darreichungsformen können die in der pharmazeutischen Industrie üblichen Träger- bzw. Verdünnungstoffe enthalten. Beispiele von geeigneten galenischen Formen sind Lösungen, Emulsionen, Suspensionen, Lotionen, Gele, Salben oder Cremes.

Die Verbindungen mit der Formel I werden bevorzugt in Kombination mit einem synthetischen oder natürlich vorkommendem Öl appliziert. Als besonders geeignet hat sich die Verwendung von pflanzlichen Ölen, insbesondere von Mandelöl, Erdnußöl, Sesamöl, Olivenöl, Weizenkeimöl, Maiskeimöl, Diestelöl, Sojaöl, Sonnenblumenöl, Kokosfett, Avocadoöl, Palmkernöl und Kakaobutter sowie auch Jojobaöl, Nachtkerzenöl, Calendula, erwiesen.

Ein weiterer Gegenstand der vorliegenden Erfindung ist eine pharmazeutische Zusammensetzung enthaltend Verbindungen mit der Formel I und ein pflanzliches ÖI oder Fett.

In einer weiteren bevorzugten Ausführungsform der vorliegenden Erfindung werden die erfindungsgemäß verwendeten Verbindungen in Kombination mit einem oder mehreren weiteren Wirkstoff(en), die eine positive, schützende und/oder pflegende Wirkung auf die Haut haben, eingesetzt. Beispiele für derartige Substanzen sind Lichtschutzfilter, Pflanzenextrakte, wie Aloe-vera- oder Kamillen-Extrakte, Biopolymere, wie Hyaluronsäure, Chitin- und Kollagen-Derivate, Bestandteile der epidermalen Lipide sowie Vitamine und Provitamine, z. B. Panthenol und α- Tocopherol. Als weitere mögliche Wirkstoffe können auch Hydroxycarbonsäuren (AHA), Liposomen und Niosomen bzw. Nanoparts genannt werden.

Die Wirkungen der Verbindungen mit der Formel I ist aus den folgenden Tests ersichtlich:

### Beispiele

Herstellung einer Salbe: 460ml Avocadoöl, 525mg Cyanocobalamin, 175mg Hydroxycobalamin, 80g Emulgator Methylglycosidstearat, 2g Kaliumsorbat, 1g wasserfreie Zitronensäure je nach pH, vermischt und anschließend mit dest. Wasser auf 1000ml aufgefüllt.

### 1. Wirkung der Prophylaxe bei neurodermitischer Disposition der Eltern

Bei einer Gruppe von 10 Kleinkindern (0-1 Jahre), deren Eltern, zumindest ein Elternteil, unter manifester Neurodermitis leiden, wurde die gemäß obigem Beispiel hergestellte Salbe 3mal täglich für den Zeitraum von 3 Monaten auf die gesamte Hautoberfläche aufgetragen. Die Wirkung der erfindungsgemäßen Salbe auf die Haut wurde für ein halbes Jahr nachbeobachtet.

Während der Nachbeobachtungszeit trat bei keinem der untersuchten Kleinkinder eine neurodermitische Hautveränderung auf.

### 2. Wirkung der Prophylaxe bei Sonnenallergie

Bei einer Gruppe von 20 Erwachsenen mit ausgeprägter Sonnenallergie in der Anamnese wurde die Hautoberfläche des Thorax und der oberen Extremitäten bei 10 Probanden für den Zeitraum von einem Monat, bei den verbleibenden 10 Probanden für den Zeitraum von 2 Monaten vor Sonnenexposition mit gemäß obigem Beispiel hergestellte Salbe einmal täglich behandelt.

Unter Sonnenexposition zeigte sich bei einem Patienten aus der einmonatig vorbehandelten Gruppe eine leichte Dermatitis solaris. Bei den anderen Probanden war eine normale Bräunung gemäß ihres Hauttyps zu beobachten.

### 3. Wirkung bei Schäden nach ionisierender Strahlung

Bei einer Gruppe von 7 Erwachsenen, die einer Strahlendosis zwischen 40 - 60 Gy ausgesetzt waren und eine akute Radiodermatitis aufwiesen, wurden die geschädigten Hautareale großflächig viermal täglich über einen Zeitraum von 14 Tagen mit der oben beschriebenen Salbe eingestrichen.

Bei 5 Patienten konnte eine vollständige Remission erreicht werden. Das Unterhautfettgewebe zeigte eine normale Struktur. 2 Patienten wiesen noch kleinere Läsionen auf, wobei auch hierbei die Besserung deutlich erkennbar war, so dass vermutet werden kann, dass eine Fortsetzung der Therapie über die 14 Tage hinaus zu einer Restitutio ad integrum führt.

### 4. Wirkung bei Verbrennung (Combustio)

Bei einem Kollektiv von 25 Patienten mit Verbrennungen 2. Grades wurde die oben beschriebene Salbe während der ersten drei Tage zweimal täglich und anschließend einmal täglich bis zur kompletten Abheilung angewendet. Bei einem Patienten mit Verbrennungen
3. Grades am rechten Unterarm wurde das Areal über einen Zeitraum von 2 Wochen zweimal täglich behandelt, danach bis zur kompletten Abheilung einmal täglich.

Die Behandlungszeit der Patienten mit Verbrennungen 2. Grades war abhängig von der Größe der geschädigten Fläche und betrug im Durchschnitt 7 Tage. Der Patient mit der Verbrennung 3. Grades benötigte 5 Wochen bis zur narbenfreien Komplettremission.

### 5. Wirkung bei Hautkrebs (Cancerosen)

Bei Untersuchungen von in-vitro- und in-vivo- Zellen konnte eindeutig ein Rückgang der Zellvermehrung der entarteten Zellen und somit einer Regression und Ausheilung bei Vorliegen eines Hautkrebses beobachtet werden.
Der Rückgang der entarteten Zellen in Zellkulturen betrug in den Zeiträumen : 2 Wochen ca 25% und 4 Wochen etwa 70%. In-vivo konnte an 7 Einzelfällen beobachtet werden, daß die Erscheinungen am Ort der Erstmanifestation nach ca. 8 Wochen nicht mehr nachzuweisen waren.

Aus den obigen Beispielen ist ersichtlich, dass die erfindungsgemäße Verwendung von Verbindungen mit der Formel I sowohl im prophylaktischen Bereich als auch auf dem Gebiet der Hautregeneration eine deutliche Wirkung besitzt.

## Patentansprüche

1. Verwendung von Corrinoiden mit der Formel I 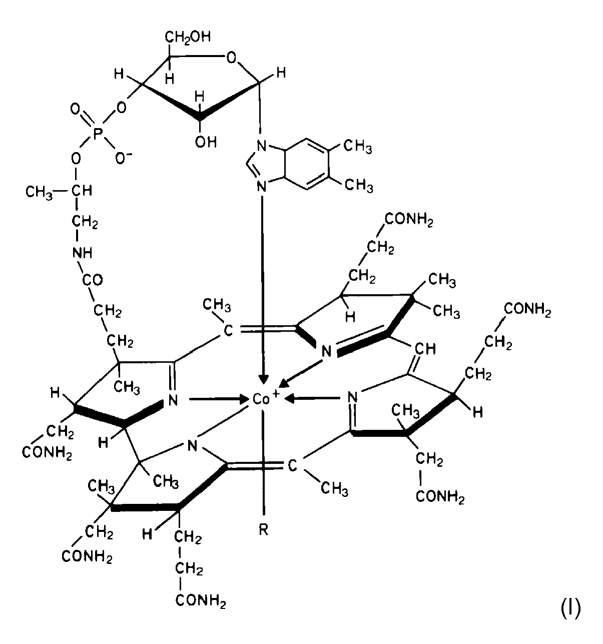 in der
R für CN, OH, CH₃ oder H₂O stehen,
in der Prophylaxe und Behandlung von allergischen Hautreaktionen.

2. Verwendung von Corrinoiden zur Herstellung eines Medikaments zur Behandlung von allergischen Hautreaktionen.

3. Verwendung nach Anspruch 1 oder 2, **dadurch gekennzeichnet, dass** die allergischen Hautreaktionen Sonnenallergien und Lebensmittelallergien sind.

4. Verwendung nach einem der Ansprüche 1 bis 3, **dadurch gekennzeichnet, dass** außerdem übliche Träger- und/oder Verdünnungsstoffe verwendet werden.

5. Verwendung nach einem der Ansprüche 1 bis 4, **dadurch gekennzeichnet, dass** zusätzliche ein oder mehrere Komponenten, die eine positive, schützende und/oder pflegende Wirkung haben verwendet werden.
